(19) Europäisches Patentamt
European Patent Office
Office européen des brevets

(11) Publication number: **0 272 810**
**A2**

(12) # EUROPEAN PATENT APPLICATION

(21) Application number: **87310411.1**

(51) Int. Cl.[4]: **C07D 403/14**, A61K 31/40

(22) Date of filing: **25.11.87**

(30) Priority: **26.11.86 US 935401**

(43) Date of publication of application:
**29.06.88 Bulletin 88/26**

(84) Designated Contracting States:
**AT BE CH DE ES FR GB GR IT LI LU NL SE**

(71) Applicant: **HARBOR BRANCH OCEANOGRAPHIC INSTITUTION, INC.**
**5600 Old Dixie Highway**
**Fort Pierce, FL 33450(US)**

(72) Inventor: **Gunasekera, Sarath P.**
**1846 23rd Place SW**
**Vero Beach FL 32962(US)**
Inventor: **Cross, Sue S.**
**1965 Jacaranda Dr.**
**Ft. Pierce FL 32962(US)**
Inventor: **Kashman, Yoel**
**5 Ruth Str.**
**Tel Aviv 64372 Israel(IL)**
Inventor: **Lui, May S.**
**6688 Paradise Lane**
**Sebastian FL 32958(US)**

(74) Representative: **Marlow, Nicholas Simon et al**
**Reddie & Grose 16, Theobalds Road**
**London WC1X 8PL(GB)**

(54) **Antitumor and antiviral alkaloids.**

(57) This invention relates to antitumor and antiviral alkaloid compositions and a process of producing the compositions. More particularly, the compositions are antitumor and antiviral bis-indole alkaloids which are derived from the marine sponge order Topsentia .

EP 0 272 810 A2

# ANTITUMOR AND ANTIVIRAL ALKALOIDS

## Field of the Invention

This invention relates to new cyclic organic compounds which have useful antiviral and antitumor activity. More particularly, this invention relates to new bis-indole alkaloid antitumor and antiviral compositions, which may be derived from marine organisms, i.e., the marine sponge, Topsentia, sp and their methods of use.

Various tumor related diseases inflict man. Considerable research has been devoted to oncology and antitumor measures. Tumors are common in a variety of mammals and the prevention, control of the growth and regression of tumors in mammals is important to man. The term tumor refers to abnormal masses of new tissue growth which is discordant with the economy of the tissue of origin or of the host's body as a whole.

Tumors inflict mammals and man with a variety of disorders and conditions including various forms of cancer and resultant cancerous cachexia. Cancerous cachexia refers to the symptomatic discomfort that accompanies the infliction of a mammal with a tumor. These symptoms include weakened condition of the inflicted mammal as evidenced by, for example, weight loss. The seriousness of cancer is well known, e.g., cancer is second only to heart and vascular diseases as a cause of death in man.

Considerable research and resources have been devoted to oncology and antitumor measures including chemotherapy. While certain methods and chemical compositions have been developed which aid in inhibiting, remitting or controlling the growth of tumors, new methods and antitumor chemical compositions are needed.

Viral diseases also inflict man, plants, insects, and animals. The prevention and control of viral diseases has important health and economic implications.

Viral diseases contribute to inflictions in humans including common colds, herpes and cancer and the importance of their control is obvious. Also important is the control of viral diseases in animals for economic reasons as well as the ability of such animals to become virus reservoirs or carriers which facilitate the spreading of viral diseases to humans. Viral plant diseases have been known to have a disruptive effect on the cultivation of fruit trees, tobacco, and various vegetables. Insect viral diseases are also of interest because of the insects' ability to transfer viral diseases to humans.

The prevention and control of viral diseases is thus of prime importance to man and considerable research has been devoted to antiviral measures. Certain methods and chemical compositions have been developed which aid in inhibiting, controlling or destroying viruses but additional methods and antiviral chemical compositions are needed.

Marine organisms and particularly marine sponges are a potential source for chemically and biologically interesting molecules of great diversity. Some such molecules derived from sponges are described in Scheuer, P.J. Ed., Marine Natural Products, Chemical and Biological Perspectives; Academic Press; New York, 1978-1983; Vol. I-V; Faulkner, D.J. Natural Products Reports 1984, Vol. 1, 551-598; and 1986, Vol. 3, 1-33 Uemura, D.; Takahashi, K.; Yamamoto, T.; Katayama, C; Tanaka, J.; Okumura, Y.; Hirata, Y. J. Am. Chem. Soc. 1985, 107, 4796-4798. The entire disclosures of these references are hereby incorporated herein by reference.

It has been desired to provide novel compositions which are useful as antiviral and antitumor agents and a process for producing such novel antitumor and antiviral compositions.

It has now been found that certain bis-indole alkaloid compositions derived from extracts of the marine sponge, Topsentia, sp. possess useful antitumor and antiviral activity.

According to the invention there is provided a composition of the general formula (I);

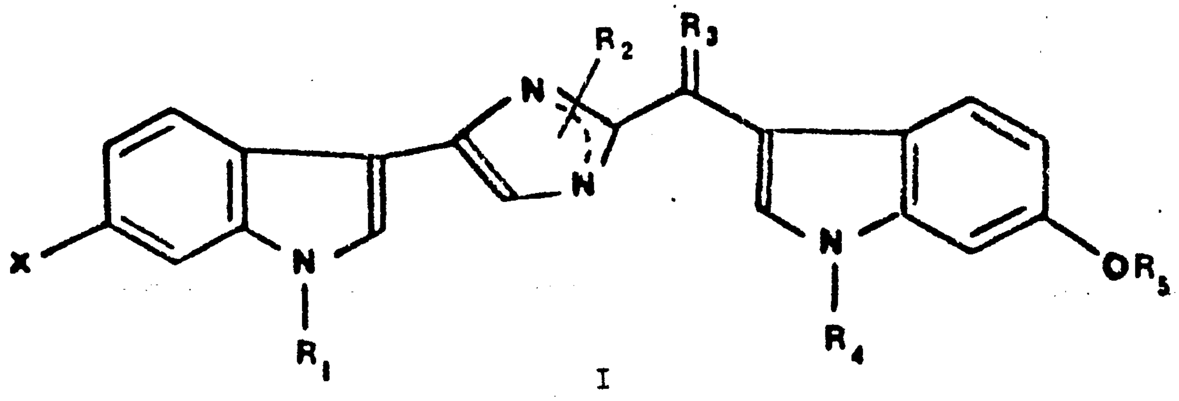

I

wherein $R^1$, $R^2$, $R^4$ and $R^5$ are the same or different and are a hydrogen, lower alkyl, or lower acyl group; $R^3$ is a double bonded oxygen or a combination of two single bonded hydrogen, hydroxy, lower alkoxy, or lower acyloxy groups which are the same or different; and X is a hydrogen, hydroxy or halogen group.

In preferred embodiments of the invention, the composition is substantially pure.

In preferred embodiments of the invention the lower alkyl, acyl, alkoxy, and acyloxy groups have from 1 to 5 carbon atoms.

In further preferred embodiments of the invention, the invention comprises a composition of the general formula I wherein X is a hydrogen or bromine and $R_1$, $R_2$, $R_3$, $R_4$ and $R_5$ have the following identities:

(1) $R_3 = H,OH$, $R_1 = R_2 = R_4 = R_5 = H$
(2) $R_3 = H,OAc$, $R_1 = R_2 = R_4 = R_5 = COCH_3$
(3) $R_3 = O$, $R_1 = R_2 = R_4 = H$, $R_5 = CH_3$
(4) $R_3 = O$, $R_1 = R_2 = R_4 = R_5 = COCH_3$
(5) $R_3 = O$, $R_1 = R_2 = R_4 = R_5 = CH_3$ .

In more preferred embodiments of the invention, the invention comprises a composition of the formula (II):

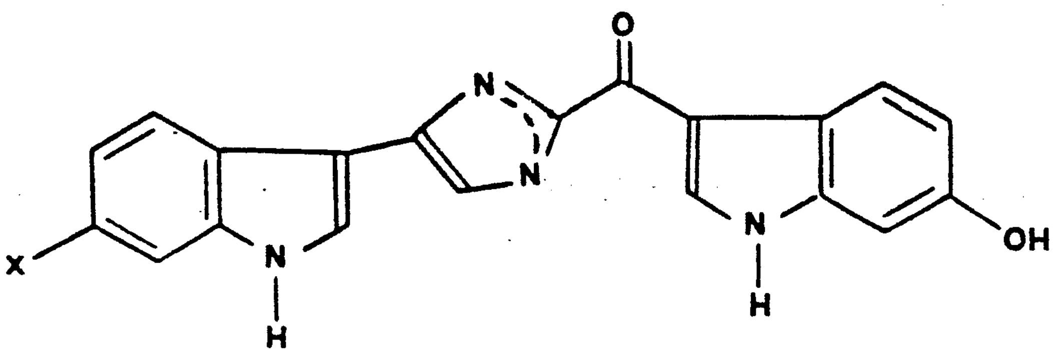

II

wherein X is hydrogen or bromine.

As embodied and fully described herein, the invention also comprises an antiviral and antitumor composition comprising, as active ingredient, an effective antitumor or antiviral amount of one or more compositions according to formulae I or II and a non-toxic pharmaceutically acceptable carrier or diluent.

As embodied and fully described herein, the invention also comprises a process to produce the compositions of formulae I and II. The process comprises the steps of collecting marine sponge Topsentia sp; contacting the sponge with at least one suitable organic solvent to obtain an extract comprising a composition according to formula I or II; and isolating a composition according to formula I or II from the extract.

In preferred embodiments of the invention the suitable organic solvent is selected from the group consisting of pentane, hexane, methanol, butanol, ethanol, toluene, acetone, methyl ethyl ketone, ethyl acetate, ethanol, and methyl isobutyl ketone.

As embodied and fully described herein, the invention further comprises a method for inhibiting tumors in a host and a therapeutic method for treating cancerous cachexia comprising contacting a tumor with an

effective antitumor amount of one or more compositions of formula I or II.

As embodied and fully described herein, the invention further comprises a method for inhibiting viruses comprising contacting a virus with an effective antiviral amount of one or more compositions according to formula I or II.

Reference will now be made in detail to present preferred embodiments of the invention, examples of which are illustrated in the following example section.

In accordance with the invention novel compositions are provided to achieve the objects in accordance with the purposes of the invention, as embodied and fully described herein, the invention comprises compositions of the general formula (I):

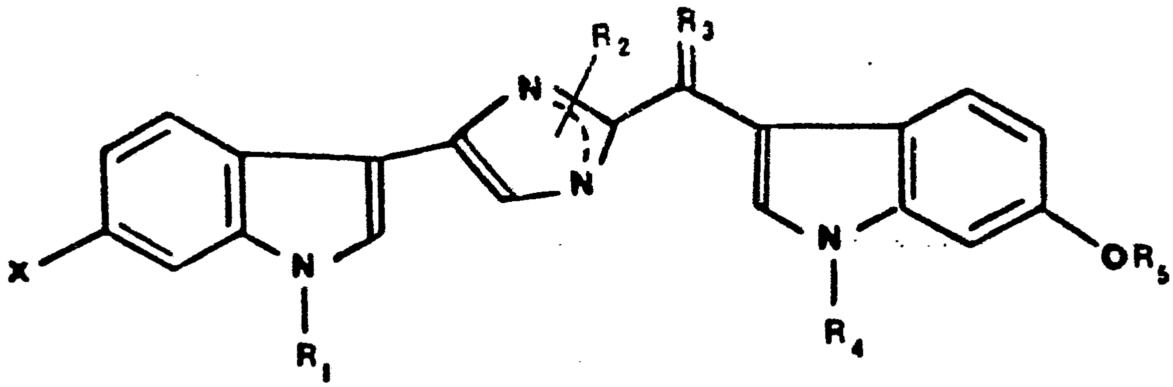

I

wherein $R^1$, $R^2$, $R^4$ and $R^5$ are the same or different and are a hydrogen, lower alkyl, or lower acyl group; $R^3$ is a double bonded oxygen or a combination of two single bonded hydrogen, hydroxy, lower alkoxy, or lower acyloxy groups which are the same or different; and X is a hydrogen, hydroxy or halogen.

In preferred embodiments of the invention the composition is substantially pure.

In preferred embodiments of the invention the lower alkyl, acyl, alkoxy, and acyloxy groups have from 1 to 5 carbon atoms.

In further preferred embodiments of the invention, the invention comprises a composition of the general formula (I) wherein X is a hydrogen of bromine, and $R_1$, $R_2$, $R_3$, $R_4$ and $R_5$ have the following identities:

(1) $R_3 = H,OH$, $R_1 = R_2 = R_4 = R_5 = H$
(2) $R_3 = H,OAc$, $R_1 = R_2 = R_4 = R_5 = COCH_3$
(3) $R_3 = O$, $R_1 = R_2 = R_4 = H$, $R_5 = CH_3$
(4) $R_3 = O$, $R_1 = R_2 = R_4 = R_5 = COCH_3$
(5) $R_3 = O$, $R_1 = R_2 = R_4 = R_5 = CH_3$

In more preferred embodiments of the invention, the invention comprises compositions of the formula (II):

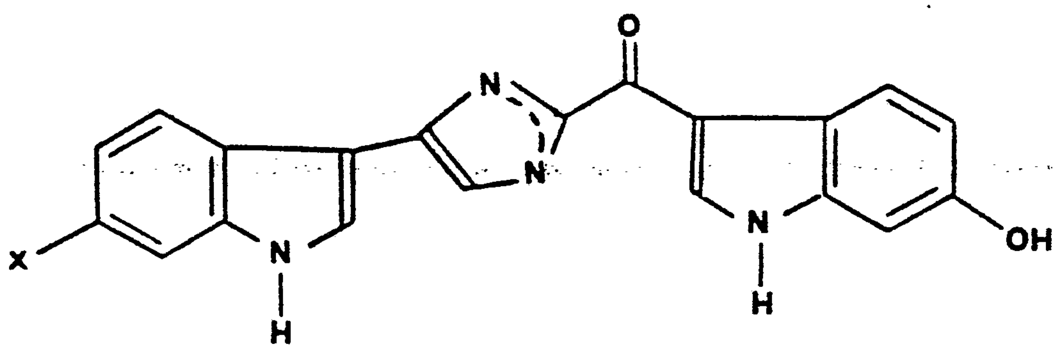

II

wherein X is hydrogen or bromine.

In accordance with the invention, an antitumor composition is provided comprising as active ingredient an effective antitumor amount of one or more of the compositions described above and identified by formulae I or II and a non-toxic pharmaceutically acceptable carrier or diluent. While effective amounts may

vary, as conditions in which the antitumor compositions are used vary, a minimal dosage required for activity is generally between 0.5 and 100 micrograms against $10^5$ tumor cells. Useful examples of non-toxic pharmaceutically acceptable carriers or diluents include, but are not limited to, the following: ethanol, dimethyl sulfoxide and glycerol.

In accordance with the invention, a method for inhibiting tumors in a host is provided comprising contacting a tumor with an antitumor amount of one or more compositions according to formulae I or II. The effectiveness of the compositions of the invention for inhibiting tumors and tumor cells indicates their usefulness for controlling tumors in hosts, including mammals, and for treating cancerous cachexia.

In accordance with the invention, an antiviral composition is provided comprising as active ingredient an effective antiviral amount of one or more of the compositions described above and identified by formula I or II and a non-toxic pharmaceutically acceptable carrier or diluent. While effective amounts may vary, as conditions in which the antiviral compositions are used vary, a minimal dosage required for activity is generally between 50 and 200 micrograms against 25-80 plaque-forming units of virus. Useful examples of non-toxic pharmaceutically acceptable carriers or diluents include, but are not limited to, the following: ethanol; dimethyl sulfoxide; and glycerol.

In accordance with the present invention, virus cells are inhibited or killed by a method comprising contacting a virus with an effective antiviral amount of one or more compositions according to formulae I or II. The minimal effective amount as stated above is generally from 50 to 200 micrograms against 25 to 80 plaque forming units of virus. The compositions of formulae I or II are active for inhibiting or killing a diverse range of viruses including, but not limited to, the RNA viruses, vesicular stomatitis (herein "VSV"), arenaviruses, coronaviruses, rhinoviruses, influenza viruses and the DNA viruses, herpes simplex-I (herein "HSV-I"), other herpes viruses, adenoviruses, coxsackie viruses, polioviruses and papovaviruses.

The effectiveness of the compositions of the invention for inhibiting virus cells indicates that the compositions of formulae I or II should also be useful in controlling viral infections in host animals and plants which are caused by a virus which is thus inhibited or destroyed. Viral infections which may be controlled by utilizing compositions of the present invention include, but are not limited to, those caused by those RNA viruses and DNA viruses described above. The invention may also be useful in controlling common viral infections of plants.

In accordance with the invention, a process to produce compositions according to formulae I or II comprises the steps of: collecting the marine sponge; contacting the sponge with at least one suitable organic solvent to obtain an organic extract comprising a composition according to formula I or II; and isolating a composition according to formula I or II.

A detailed description and explanation of a preferred embodiment of the process of the invention to produce the compositions according to formula I or II and their reduced or acid salt derivatives is as follows: the marine sponge Topsentia, sp. is collected by SCUBA at a depth of 1149 feet at Goulding Cay, Bahamas. The marine sponge is extracted with methanol-toluene (3:1) as a first solvent to obtain an extract which is concentrated to yield a crude residue. The residue is then partitoned between pentane and 10% aqueous methanol as first partitioning solvents to obtain an aqueous methanol extract. The aqueous methanol layer is then diluted to 30% water and the residue is partitioned between 30% aqueous methanol and dichloromethane as second partitioning solvents. The aqueous methanol layer is then concentrated and the residue is partitioned between butanol and water as third partitioning solvents. The butanol soluble fraction, which comprises a composition according to formula I or II, is dissolved in 20% aqueous methanol and the substantially pure composition of formula I or II is obtained by various chromatographic methods.

While methanol-toluene is the presently preferred choice for the first extracting solvent, other suitable solvents may be substituted. A suitable solvent should be capable of extracting a compound according to any one of formula I or II from other compounds of the marine sponge. Suitable first, second and third partitioning solvents may be substituted for pentane, dichloromethane, butanol and methanol. For example, other alkanes $C_5$-$C_{10}$ may be substituted for pentane and other lower alkanols may be substituted for butanol or methanol.

Any suitable fractionation and isolation techniques may be utilized in accordance with the process of the invention. Suitable fractionation techniques include various chromatography techniques such as reverse phase and high pressure liquid chromatography (RPLC and HPLC, respectively) with a suitable column as would be known to those skilled in the art including silica gel; sephadex LH-20; ammonia-treated silica gel; and LiChrosorb $NH_2$ columns. These columns are eluted with suitable eluents such as: heptane; ethyl acetate; methylene chloride; methanol; isopropyl alcohol; and various combinations and ratios thereof as would be known to those skilled in the art.

EXAMPLES

The invention will now be illustrated by examples. The examples are not intended to be limiting of the scope of the present invention. In conjunction with the detailed and general description above, the examples provide further understanding of the present invention and outline a process for producing compositions of the invention.

The following examples represent preferred embodiments of the compositions, processes and methods of the invention for satisfying the stated objects of the invention. The starting materials and reagents in the examples whose method of preparation are not included are commercially available from sources known to the art such as chemical supply houses.

Examples 1 and 2

The antitumor and antiviral bis-indole alkaloids of the invention were prepared from a marine sponge, Topsentia sp., according to the following procedures.

Preparation of Topsentin and Bromotopsentin:

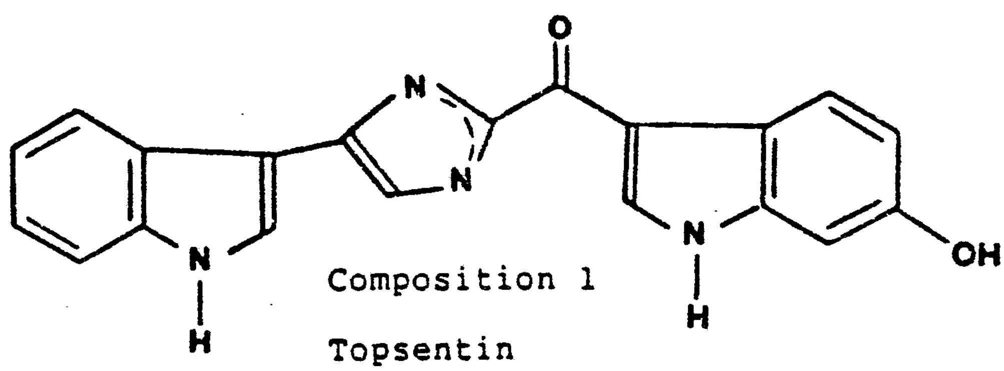

Composition 1

Topsentin

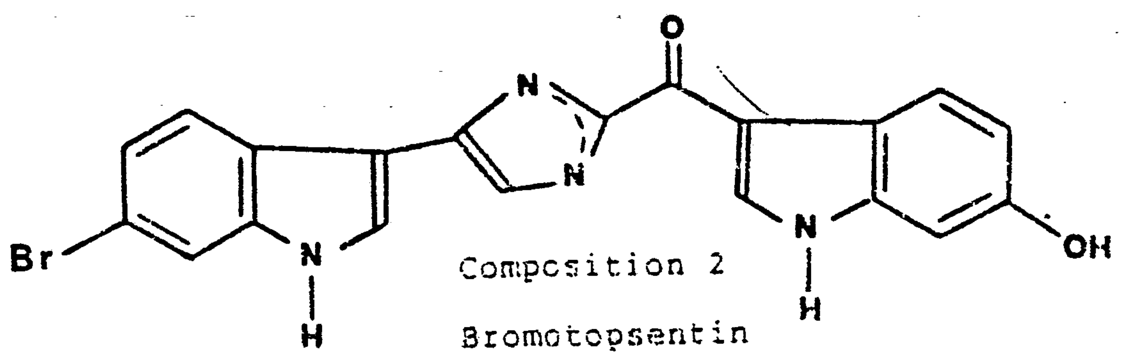

Composition 2

Bromotopsentin

The frozen sample (264 g), which was collected at a depth of 1149 feet at Goulding Cay, Bahamas was extracted twice with methanol-toluene (3:1). The combined extracts on concentration on a water bath at 30°C in vacuo gave as a residue 11.32 g. of the crude extract. The residue (11.32 g.) was partitioned between pentane and 10% aqueous methanol. The alcohol layer was then diluted to 30% water and extracted with $CH_2Cl_2$. The aqueous methanol layer was concentrated and partitioned between butanol and water. A portion (200 mg) of the Herpes simplex virus type 1 (HSV-1) active butanol soluble fraction was dissolved in 20% aqueous methanol (1 ml) and chromatographed on a column (ID = 22 mm, height = 40 mm) packed with reverse phase material (Amicon silica - C8, 20-45 um). The active fraction (123 mg.) was eluted with 20% aqueous methanol and purified by reverse phase HPLC (IBM 5u C18, 10 mm x 250 mm, 20% aqueous methanol) to yield pure topsentin (1), 28 mg, and bromotopsentin (2), 67 mg. as yellow powder.

Topsentin (1), yellow powder; UV absorption, λ max (MeOH) 208 nm (12,000), 246 sh, (5100), 285

(4500) and 375 (4600); IR (KBr) 3395, 3275, 1635, 1590, 1530, 1455, 1270, 1165, 1115, 1095, 1005, and 876 $cm^{-1}$; $^1$HNMR (360 MHz, DMSO-$d_6$ + 1% TFA-H) 6.841 (1H, dd, J = 8.6, 1.8 Hz), 7.997 (1H, d, J = 1.8 Hz), 7.201 (2H, m), 7.523 (1H, d, J = 7.9 Hz), 7.990 (1H, d, J = 7.6 Hz, 8.041 (1H, d, J = 8.6 Hz), 8.155, (1H, d J = 2.8 Hz), 8.159 (1H, s), 8.487 (1H, d, J = 3.2 Hz), 11.762 (1H, s), 12.355 (1H, d, J = 2.2 Hz). $^{13}$C NMR (90 MHz, DMSO + 1%TFA-H) 98.11 (d), 102.72(s), 112.38(d), 113.12 (d), 113.95 (s), 116.00 (d), 118.67 (s), 119.46 (d), 120.50 (d), 122.02 (d), 122.44 (d), 124.27 (s), 125.74 (d), 131.11 (s), 136.53 (s), 137.78 (d), 138.33 (s), 141.23 (s), 155.25 (s), 171.5 (s); HREIMS 342 (100%, $C_{20}H_{14}N_4O_2$, M ), 209 (39, $C_{12}H_7N_3O$), 183 (28, $C_{11}H_9N_3$), 171 (17, $C_{10}H_7N_2O$), 160 (145, $C_9H_6NO_2$) 133(65, $C_8H_7NO$) and 105 (15).

Bromotopsentin (2), yellow crystals, mp 296-7°C; UV absorption,λmax (MeOH) 209 nm (13,000), 236 (9,700), 287 (5000) and 374 (5800); IR (KBr) 3400-3100, 2255, 2120, 1635, 1590, 1520, 1445, 1265, 1230, 1165, 1028, 1005, and 875 $cm^{-1}$; $^1$H NMR (360 MHz, $CDCl_3$:$CF_3COOH$ 1:1) 7.098 (1H, dd, J=8.6, 2.4 Hz), 7.193 (1H, d, J=2.4 Hz), 7.227 (1H, dd, J=8.6, 1.8 Hz), 7.558 (1H, d, J=8.6 Hz), 7.668 (1H, d, J=1.8 Hz), 7.824 (1,s), 7.927 (1H, d, J=3 Hz), 8.202 (1H, d, J=8.6 Hz), 8.371 (1H, d, J=3 Hz), 9.272 (1H, brs), 10.409 (1H, brs); $^{13}$C NMR (90 MHz, $CDCl_3$:$CF_3$:COOH 1:1) 101.6 (d), 103.7 (s), 116.7 (d), 117.0 (s), 117.6 (d), 118.2 (d), 119.6 (s), 121.5 (d), 122.6 (s), 125.2 (s), 125.5 (d), 127.7 (d), 128.0 (d), 135.0 (s), 139.7 (s), 140.5 (s), 140.8 (d), 141.7 (s), 155.0 (s), 172.4 (s); High resolution mass spectrum (HREIMS) 422/420 (40%, $C_{20}H_{13}BrN_{14}O_2$, M ), 394/392 (1.3, $C_{19}H_{11}BrN_3O_2$), 342 (13, M -Br), 289/287 (6%, $C_{12}H_7BrN_3O$), 263/261 (100, $C_{11}H_8BrN_3$), 223/221 (13, $C_9H_6BrN_2$), 209/207 (9.5, $C_9H_6BrNO$), 182 (15, 261-Br) and 133 (94, $C_8H_7NO$).

## ANTITUMOR ACTIVITIES OF THE COMPOSITIONS OF THE INVENTION

The following assay method was utilized to illustrate the antitumor effectiveness of the compositions of formulae I and II, corresponding to compositions 1 (topsentin) and 2 (bromotopsentin) of the examples.

## P388 MOUSE LEUKEMIA CELL ASSAY

### Maintenance of Cell Line

P388 mouse leukemia cells are grown in Dulbecco MEM medium with 10% horse serum, 4mM glutamine, and 20ug/ml gentamycin (Biologos, Inc.). Cells are incubated in 10% $CO_2$ and subcultured 2 times per week.

### PROCEDURE

1. Add compound to each well of a 24-well plate or tube and allow solvent to evaporate to dryness.
2. Add 2ml (1.2 x $10^5$) cells to each well or tube and mix.
3. Incubate in 10% $CO_2$ at 37° for 48 hours.
4. Read plates with an inverted microscope, scoring activity from 1+ to 4+ as follows: ND (not detectable), >90%; 1+, 75-90%; 2+, 50-74%; 3+, 25-49%; 4+, <25% of control growth. Cell counts are performed on each tube and results are reported as percent of control.

Positive control - Vinblastine or Vincristine in aqueous solution.

## HUMAN TUMOR CELL LINE ASSAY

### Maintenance of Cell Lines

HCT-8 human colon tumor cells are grown in RPMI 1640 medium (GIBCO). A549 human lung carcinoma cells and T47D human breast carcinoma cells are cultured in Dulbecco medium (Biologos, Inc.). All media are supplemented with 10% fetal bovine serum and contain 50 ug/ml gentamycin. All human tumor cell lines are incubated in 5% $CO_2$ at 37° and subcultured once a week.

PROCEDURE

1. Seed 1ml of cells (5000 HCT-8, 8000 A549, 12000 T47D) into each well of a 24-well plate.
2. Incubate in a $CO_2$-incubator for 48 hours.
3. Add compound to each well and incubate for an additional 120 hours.
4. Discard medium and stain with methylene blue (HCT-8) or crystal violet (A549 and T47D).
5. Compare cell density of drug-treated well with that of the control (no drug added) as follows: ND (not detectable), >90%; 1+, 75-90%; 2+, 50-74%; 3+, 25-49%, 4+, <25% of control growth.

Final Conc. of Vinblastine or Vincristine control (use 2 ul/assay)

| Solution Conc. | Amt added | Final conc. in test |
|---|---|---|
| 50 mg/ml | 2 ul | 50 ug/ml |
| 20 mg/ml | 2 ul | 20 ug/ml |
| 5.0 mg/ml | 2 ul | 5.0 ug/ml |
| 0.5 mg/ml | 2 ul | 0.5 ug/ml |

Notes:

1. For solvents other than water, allow solvent to evaporate from tube or well in hood.
2. Chloroform and butanol cannot be used in the plastic 24-well plates - use glass tubes.

Always run a solvent control in duplicate in the last two wells of each plate or four tubes for each rack of 72 or less tubes. Also run four wells or tubes with media or cells only per run of plates or tubes. When using volumes of aqueous solutions greater than 200 ul, dry sample and bring up to desired concentration in media.

The results of the above assay are summarized in Table 1.

Table 1: Antitumor Assay Results of In Vitro Composition 1 and 2

| | P388 ug/ml | | | | HCT-8 ug/ml | | | | A-549 ug/ml | | | | T47D ug/ml | | | |
|---|---|---|---|---|---|---|---|---|---|---|---|---|---|---|---|---|
| Composition | 50 | 20 | 5 | 0.5 | 50 | 20 | 5 | 0.5 | 50 | 20 | 5 | 0.5 | 50 | 20 | 5 | 0.5 |
| Composition 1 | 4+ | 4+ | 2+ | ND | 4+ | 4+ | 1+ | ND | 4+ | 4+ | ND | ND | 4+ | 4+ | 2+ | ND |
| Composition 2 | 4+ | 4+ | ND | ND | 4+ | 4+ | ND | ND | 4+ | 4+ | ND | ND | 4+ | 3+ | 2+ | ND |

Table 1 shows that compositions 1 and 2 have good antitumor activity at concentrations of at least 20 ug/ml.

Procedure for P388 In-vivo Experiments

P388 leukemia obtained from DBA/2 mice was inoculated ip into BDFI mice. The inoculum level was $10^6$ cells in 0.1 ml. Mice were randomized on day 1 (24 hrs post-inoculation) into groups of six mice since bacteriological check of tumor was negative. Test materials were dissolved or suspended in sterile 0.9% NaCl

solution with the aid of absolute ethanol and Tween-80, then administered ip, qD1-5, in a volume of 0.5 ml/mouse. Mice were weighed on days 1 and 5 to provide evidence of toxicity and deaths were recorded daily. Each experiment included appropriate numbers of untreated control mice, one-dose level of the positive reference compound 5-fluorouracil, and test materials (four-dose levels each). Test materials were prepared fresh on day 1 and administered daily for five days. Quantity and consistency of test materials precluded fresh preparation daily. Doses were derived from prior single treatment acute toxicity assays. The endpoints for therapeutic evaluation were mean and median survival time and long-term survivors on day 30. Percent increase in life span (% ILS) 25 was considered evidence of significant activity.

In vivo testing against ip P388 lymphocytic leukemia

Table 2: Antitumor Assay Results In Vivo of Compositions 1 and 2

| Dose mg/kg | Treatment Schedule (Days) | Median Survival % T/C |
|---|---|---|
| Composition 1 (Topsentin) | | |
| 300 | 1 - 5 | 110 |
| 150 | 1 - 5 | 132 |
| 75 | 1 - 5 | 116 |
| 37.5 | 1 - 5 | 111 |
| Composition 2 (Bromotopsentin) | | |
| 300 | 1 - 4 | toxic |
| 150 | 1 - 5 | 116 |
| 75 | 1 - 5 | 111 |
| 37.5 | 1 - 5 | 105 |

It is apparent from the in vitro and in vivo testing and results reported in Tables 1 and 2 that the compositions of the invention are effective for inhibiting or destroying tumors and therefore in controlling diseases caused by or related to such tumors such as cancerous cachexia in fulfillment of the objects of the invention.

ANTIVIRAL ACTIVITIES OF THE COMPOSITIONS OF THE INVENTION

The following assay method was utilized to demonstrate the in vitro antiviral effectiveness of the compositions of formulae I and II, corresponding to compositions 1 and 2 (topsentin and bromotopsentin, respectively) of the examples.

Antiviral Disc Assay for HSV-1

A. Maintenance of Cell Cultures

1. Virus

a. Herpes simplex type 1 (HSV-1) replicates in the CV-1 cell line. CV-1 is a fibroblast-like cell culture derived from primary African green monkey cells.

2. Growth of CV-1 Cells

a. Seed $150cm^2$ tissue culture flasks each with $10 \times 10^6$ CV-1 cells in 40 ml of EMEM with 10% FBS (growth medium).

b. Seven days after seeding the flasks cell numbers should be approximately $40\text{-}50 \times 10^6$ cells. CV-1 cells have a doubling time of 72 hours based on these numbers.

3. Trypsinization

a. Aseptically remove the medium.

b. Rinse cell sheet two times with 10 ml of Ca and Mg free Dulbecco's phosphate buffered saline.

c. Add 1.5 to 2.0 ml of trypsin - EDTA mixture.

d. Incubate flask at room temperature for 10 minutes.

e. Shake flask.

f. Add 10 ml EMEM growth medium and break up cell clumps with pipetting.

g. Count cells.

B. Preparation of plates for viral assays

1. Cell Concentration

a. Dilute the cells with EMEM to $4 \times 10^5$ cells/ml.

b. Seed 24 well trays with 0.5 ml per well. Cell concentration per well is $2 \times 10^5$ cells.

c. Incubate at 37°C with 5% $CO_2$.

d. The wells can be uses over the next several days beginning the day after seeding (preferably 2, 3, or 4).

C. Assay of HSV-1 in CV-1 cells

1. Infection of CV-1 cells in plates with virus

a. Remove medium from wells.

b. Infect well with at least 25 and no more than 80 plaque forming units (PFU) of virus.

c. Incubate infected cells at 37°C for 1.5 hours.

d. Pour off supernatant at end of incubation period.

e. Add 0.5 ml of methylcellulose overlay medium (MCO).

1) MCO is a maintenance medium without phenol red made with a 4000 centipose methylcellulose. FBS is used at 5% level.

2. Drug Evaluation

a. For drug evaluation wet filter paper discs (6 mm diameter) with approximately 0.02 ml of marine extract or test compound.

1) Allow solvent to evaporate for 20 to 30 minutes at room temperature.

2) Place discs in the well containing CV-1 cells, virus, and MCO.

b. Incubate tissue culture plates for 48 hours at 37° C.

c. After 48 hours place 0.5 ml NRMCO on each well.

1) NRMCO is a maintenance overlay medium without phenol red containing 0.1 mg neutral red dye per ml and 2% 15 centipose methylcellulose.

d. Incubate plates at 37°C and read the following day.

1) Antiviral activity should be observed from two parameters. One is actual reduction in the number of plaques and two is the diminution in plaque diameter.

3. Scoring Drug Activity

a. Antiviral activity (AVA) is scored from 0 to + + +.

+ + + = complete inhibition of plaque formation

+ + = partial inhibition

+ = partial inhibition

0 = no protection

b. Cytotoxicity (Cyt)

0 = no visual or microscope cytotoxicity

16 = Complete cell destruction

8, 10, 12, 14 = partial cytotoxicity

The results of the above assay are summarized in Table 3.

Table 3: Antiviral Activity of Compositions 1 and 2.

| Composition | Dose (ug/well) | HSV-1 Cyt. | HSV-1 AVA |
|---|---|---|---|
| 1 | 200 | 0 | ++ |
| | 100 | 0 | + |
| 2 | 200 | 0 | ++ |

Table 3 shows that composition 1 and 2 have antiviral activity at concentrations of 100 mg/ml and/or 200 mg/ml.

It is apparent from the <u>in vitro</u> testing that the compositions of the invention are effective for inhibiting viral growth and therefore for controlling virus related diseases such as herpes and the common cold, in fulfillment of the objects of the invention.

The scope of the present invention is not limited by the description, examples, and suggested uses herein and modifications can be made without departing from the spirit of the invention. For example, it may be noted that other derivatives of the compositions of examples 1 and 2 such as acyl or fluorinated derivatives may possess antitumor and antiviral activity analogous to those preferred embodiments described above. Further, the compositions described herein may have other useful applications such as, for example, analgesic applications. Applications of the compositions of the present invention can be accomplished by any suitable therapeutic method and technique presently or prospectively known to those skilled in the art. Thus, it is intended that the present invention cover the modifications and variations of this invention provided that they come within the scope of the appended claims and their equivalents.

## Claims

1. A composition according to the general formula:

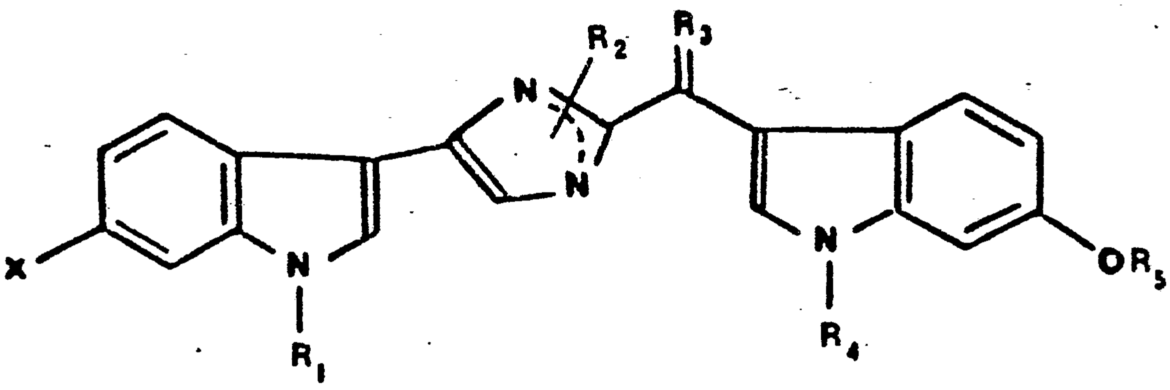

wherein $R^1$, $R^2$, $R^4$, and $R^5$ are the same or different and are hydrogen, lower alkyl, or lower acyl; $R^3$ is oxygen or two single bonded hydrogen, hydroxy, lower alkoxy, or lower acyloxy which are the same or different; and X is hydrogen, hydroxy or halogen.

2. A composition according to claim 1 in which X is hydrogen or bromine and:

(a) $R_1$, $R_2$, $R_4$, and $R_5$ are hydrogen and $R_3$ is oxygen;
(b) $R_1$, $R_2$, $R_4$ and $R_5$ are hydrogen and $R_3$ is hydrogen and hydroxy;
(c) $R_1$, $R_2$, $R_4$ and $R_5$ are $-COCH_3$ and $R_3$ is hydrogen and -OAc;
(d) $R_1$, $R_2$ and $R_4$ are hydrogen, $R_5$ is $-CH_3$ and $R_3$ is oxygen;
(e) $R_1$, $R_2$, $R_4$ and $R_5$ are $-COCH_3$ and $R_3$ is oxygen;
(f) $R_1$, $R_2$, $R_3$, $R_4$ and $R_5$ are $-CH_3$ and $R_3$ is oxygen.

3. A composition according to claim 1 or 2 in which the composition is substantially pure.

4. An antitumor composition comprising, as active ingredient, an effective antitumor amount of one or more of the compositions of claim 1, 2 or 3 and a non-toxic pharmaceutically acceptable carrier or diluent.

5. An antiviral composition comprising, as active ingredient, an effective antiviral amount of one or more of the compositions of claim 1, 2 or 3 and a non-toxic pharmaceutically acceptable carrier or diluent.

6. A pharmaceutical composition comprising one or more of the compositions of claims 1, 2 or 3 and a non-toxic pharmaceutically acceptable carrier or diluent.

7. A process to produce a composition according to any preceding claim comprising the steps of:
collecting marine sponge Topsentia ;
contacting the said sponge with a suitable organic solvent;
obtaining an extract of the sponge and solvent mixture; and
isolating a composition according to claim 1, 2 or 3 from the extract.

8. A method of inhibiting tumors in a host comprising contacting a tumor with an effective antitumor amount of one or more compositions according to any of claims 1 to 6.

9. A method for inhibiting tumors according to claim 8 in which the host is a mammalian host.

10. A method for inhibiting viruses in a host comprising contacting a virus with an effective antiviral amount of one or more compositions of any of claims 1 to 6.

11. A therapeutic method for treating cancerous cachexia caused by the presence of a tumor in a host comprising contacting cells of the tumor with an effective antitumor amount of a composition according to any of claims 1 to 6.